(19) **Europäisches Patentamt**

**European Patent Office**

**Office européen des brevets**

(11) Numéro de publication : **0 074 890**
**B1**

(12) **FASCICULE DE BREVET EUROPÉEN**

(45) Date de publication du fascicule du brevet :
07.08.85

(51) Int. Cl.⁴ : **G 01 N 33/76, G 01 N 33/555**

(21) Numéro de dépôt : **82401630.7**

(22) Date de dépôt : **03.09.82**

(54) **Procédé pour la détection de quantités très minimes d'antigènes et d'anticorps par un test amélioré d'inhibition d'agglutination.**

(30) Priorité : **15.09.81 US 302589**

(43) Date de publication de la demande :
**23.03.83 Bulletin 83/12**

(45) Mention de la délivrance du brevet :
**07.08.85 Bulletin 85/32**

(84) Etats contractants désignés :
**AT BE CH DE FR GB IT LI NL SE**

(56) Documents cités :
**DE-A- 2 703 523**
**DE-A- 2 736 684**
**DE-A- 2 743 445**
**DE-A- 2 745 279**
**US-A- 3 541 202**
**US-A- 3 843 777**
**W.D. ODELL, W.H. Danghaday: "Principles of Compe-**
**titive protein binding assays" page 43 J.B. Lippincott**
**Co.**

(73) Titulaire : **ANDA BIOLOGICALS**
**37, rue de la Course**
**F-67000 Strasbourg (FR)**

(72) Inventeur : **Maes, Roland F.**
**10A, rue du 22 Novembre**
**F-67000 Strasbourg (FR)**

(74) Mandataire : **Beauchamps, Georges et al**
**Cabinet Z.Weinstein 20, avenue de Friedland**
**F-75008 Paris (FR)**

## Description

La présente invention concerne un procédé pour la détection de quantités très minimes d'antigènes et d'anticorps par un test amélioré d'inhibition d'agglutination.

On sait que la détermination, dans les liquides, de substances ayant une valeur diagnostique ou clinique au moyen d'une agglutination ou inhibition de l'agglutination d'une phase solide sensibilisée telle que des globules rouges, des particules colloïdales, du latex ou des bactéries, est connue depuis plus de vingt ans. Le test de diagnostique est exécuté de la manière suivante : les billes sont activées par un agent pontant (acide tannique, glutaraldéhyde, difluoro-dinitro-benzène, etc...), et sensibilisées par un antigène ou anticorps. La substance sensibilisante peut aussi s'absorber spontanément aux billes.

La phase solide sensibilisée est mélangée avec l'antigène soluble ou l'anticorps à déterminer ainsi qu'avec une quantité déterminée de l'antigène ou de l'anticorps spécifique. En l'absence d'antigène soluble, la phase solide granulée sensibilisée par l'antigène est agglutinée ou agrégée par l'anticorps. En présence d'un antigène soluble ajouté, cet antigène se liera à l'anticorps, et ainsi inhibera l'agglutination ou l'agrégation de la phase solide.

De la même façon, une phase solide granulée sensibilisée par un anticorps agrégera ou agglutinera en présence d'une quantité déterminée d'antigène et cette agrégation sera inhibée par un anticorps soluble additionnel.

Ce système est très sensible mais il a été, ces dernières années, remplacé par des techniques plus raffinées, parmi lesquelles les tests de détection radioimmunologique sont prépondérants. Dans ces tests de détection radioimmunologique, on peut faire prolonger la réaction antigène-anticorps afin d'augmenter la valeur finale d'antigène lié, et ainsi d'augmenter la sensibilité. Cependant, les tests radioimmunologiques exigent des radio-éléments, des instruments de détection coûteux et une main-d'œuvre hautement qualifiée.

Il n'est donc pas inintéressant de chercher à améliorer la sensibilité du test basé sur l'agglutination d'une phase solide et sensibilisée, de façon à pouvoir concurrencer les tests radioimmunologiques.

La présente invention a donc pour objet un procédé pour la détection d'antigènes, tels l'hormone lutéinisante (LH), l'hormone gonadotropine chorionique (HCG), l'alpha féto-protéine (AFP), la progestérone, l'antigène carcino-embryonique, la testostérone, la phosphatase acide et d'autres haptènes tels que l'héroïne, la cocaïne, l'acide lysergique et ses dérivés, ainsi que leurs anticorps. La raison principale de l'absence d'un test d'agglutination simple et fiable pour la détection de ces structures est leur faible concentration dans le plasma, l'urine, le liquide céphalorachidien et autres. Pour illustrer cette situation et sans que cet exemple soit en rien limitatif, nous analyserons ici le cas de l'hormone lutéinisante (L.H.) qui est un indice de l'ovulation chez les mammifères.

Chez la femme, la L.H. est sécrétée normalement au quatorzième jour du cycle menstruel mais cette hormone est sécrétée également en petites quantités en dehors du pic ainsi que par les hommes. Cette hormone est très similaire à l'hormone de grossesse (l'hormone gonadotropine chorionique) sécrétée par l'être humain à partir du moment de la fécondation et aussi par certaines tumeurs malignes.

Ces deux hormones protéiniques possèdent la même sous-unité α et diffèrent légèrement dans la nature de leur sous-unité β, qui est plus longue d'une vingtaine d'acides aminés chez l'H.C.G.

Des tests de grossesse détectant les sous-unités α et β de l'H.C.G. sont bien connus. Ces tests immunologiques, ainsi que des tests plus spécifiques basés sur l'utilisation d'anticorps contre le L.H. pourraient être utilisés pour la détection de la L.H. Egalement des tests de grossesse spécifiques pour la sous-unité β de l'H.C.G. pourraient être utilisés pour la détection des tumeurs sécrétant la sous-unité β de l'H.C.G. Cependant, pour l'instant, seuls les tests radio-immunologiques compliqués et coûteux sont utilisés pour la détection de quantités infimes de L.H. ou de β-H.C.G.

Le motif pour lequel des tests d'agglutination et d'agrégation ne sont pas utilisés est la quantité très faible d'hormone lutéinisante sécrétée au moment de l'ovulation, ainsi que le niveau très bas des marqueurs tumoraux sécrétés par les cancers au moment de leur éclosion, c'est-à-dire, précisément au moment où il est important de les détecter. Des tests d'agglutination conventionnels ne sont pas assez sensibles pour les détecter.

Un test immunologique conventionnel d'agglutination pour la détection de la β-H.C.G. ou de la L.H. est utilisé de la façon suivante : de l'urine ou autre fluide corporel contenant la substance à déterminer est ajouté à un mélange de cellules indicatrices sensibilisées (en règle générale des érythrocytes de mouton sensibilisées par l'antigène) ainsi qu'un anticorps spécifique dilué de façon appropriée. Si le fluide analysé est dépourvu d'antigène, les anticorps présents dans le mélange agglutinent les globules rouges et ceux-ci sédimentent en un tapis uniforme au fond du tube de sérologie ou au fond de la cupule.

Si le liquide contient de l'antigène, cet antigène réagira sur l'anticorps solubilisé et une inhibition de l'agglutination va se produire. Les cellules sédimentent alors librement au fond du tube en un anneau compact. Un test de ce genre, appliqué à l'antigène australien est décrit par Vyas (U.S. patent 3 887 697).

La réaction antigène-anticorps est très lente à 4 °C et le test IHA (inhibition de l'hémoagglutination) est en général conduit à température ambiante (T.A.) ou à 37 °C. Lorsque les réactifs utilisés ont été stockés dans un réfrigérateur, ils sont en général ramenés à T.A. avant l'addition du système indicateur. Un tel système est décrit dans « Methods in Enzymology », vol. 70, Immunochemical Techniques, page 462-463, Academic Press.

Le système est très sensible et a donné lieu à plusieurs applications particulières, telles celles décrites par Vyas et al. in U.S. patent 3 887 697 et encore Mochida et al. U.S. patent 4 308 026.

Ce procédé permet la détection d'environ 125 unités internationales d'H.C.G. par litre de liquide ou encore de 275 unités internationales de L.H. par litre.

Etant donné que les tumeurs naissantes sécrètent des quantités infimes de β-H.C.G. (détectées par un test radio-immunologique spécifique de la sous-unité β), d'une part, et que le pic de sécrétion de L.H. au 14e jour suivant le début de la période menstruelle est en moyenne de 40 unités internationales de L.H. par litre, les tests d'inhibition de l'agglutination disponibles pour la détection de β-H.C.G. ou de L.H. sont trop peu sensibles pour la détection de l'ovulation chez une femme normalement cyclée ou pour la détection du marqueur tumoral β-H.C.G., au moment de la naissance de la tumeur.

Il a été trouvé de façon surprenante selon la présente invention que la limite de sensibilité d'un test d'inhibition de l'agglutination pouvait être améliorée d'un facteur 10 à 20 simplement en retardant le moment auquel le système indicateur, de préférence les globules rouges sensibilisés, est ajouté au mélange anticorps-antigène. Il a été trouvé que les protéases habituellement présentes dans les liquides analysés (plasma, sérum, urine, liquide céphalo-rachidien) ne sont pas suffisamment actives pour interférer de façon significative avec la réaction antigène-anticorps durant le temps nécessaire pour que cette réaction soit achevée.

En d'autres termes, la présente invention a pour objet un procédé pour la détection d'antigènes et d'anticorps par un test d'inhibition d'agglutination, selon un mécanisme immunologique du type réaction antigène-anticorps, comprenant une réaction des anticorps avec les antigènes dans une phase liquide dans des conditions de réaction entre les antigènes et les anticorps, par une réaction immunologique primaire, et l'ajout d'un système indicateur approprié, caractérisé en ce que ledit système indicateur est un système indicateur sensibilisé que l'on ajoute avec un retard pouvant aller de 15 minutes, de préférence une heure, à 18 heures après que la réaction antigène-anticorps ait eu lieu.

Selon un mode de réalisation particulier de ce procédé, le système indicateur sensibilisé est une phase solide, granulée, sensibilisée. De préférence, le système indicateur sensibilisé est une phase solide granulée sensibilisée à l'antigène. De préférence, cette phase solide granulée est composée soit d'érythrocytes sensibilisés soit consiste en bactéries sensibilisées.

De même, selon un mode de réalisation particulier, les anticorps spécifiques sont lyophilisés en quantités déterminées dans des tubes individuels et l'antigène peut également être avantageusement lyophilisé en quantités déterminées dans des tubes individuels.

De plus, pour que le système gagne en fiabilité, il a été trouvé que le réactif contenant les anticorps peut contenir des inhibiteurs de protéases et des bactériostatiques et bactéricides, tels que l'azide de sodium, le merthiolate, le trasylol, le di-isopropyl-fluorophosphate (DFP), de l'EDTA et autres substances qui inhibent l'activité des bactéries et des protéases durant le temps où la réaction immunologique a lieu, sans toutefois la mettre en danger.

Typiquement, une quantité prédéterminée d'anticorps contre l'antigène ou haptène à déterminer (par exemple β-H.C.G., F.P., C.E.A., héroïne) est diluée dans un réactif adapté et fournie sous forme liquide ou encore lyophilisée pour une plus grande stabilité au cours du stockage. A ces anticorps spécifiques est ajouté le liquide (en général 0,1 à 0,15 ml) contenant l'antigène ou haptène à déterminer. Au lieu d'ajouter immédiatement à ce mélange (porté à température ambiante si nécessaire) le système indicateur sensibilisé (bactéries sensibilisées, latex sensibilisé, érythrocytes sensibilisés, etc...) et faire la lecture de la réaction soit immédiatement sous forme d'une agrégation soit après le dépôt de la phase solide au fond d'un tube, l'addition du système indicateur est retardée. Antigènes et anticorps spécifiques en phase liquide sont autorisés à réagir entre eux à une température atteignant en général 20 à 25 °C. Le laps de temps durant lequel cette réaction primaire a lieu avant l'addition du système indicateur définit le niveau de sensibilité du test. Le système indicateur consiste en une phase solide granulée sensibilisée par un antigène ou un anticorps.

Les exemples suivants illustrent l'utilité de l'invention sans qu'ils soient cependant limitatifs :

Exemple 1

L'urine d'homme est complétée par de l'H.C.G. à des concentrations variant de 200 UI/l à 7,5 UI/l. Des anticorps contre la sous-unité β de l'H.C.G. obtenus par l'inoculation de la sous-unité β à des lapins, sont dilués de façon à obtenir, dans un test de l'inhibition de l'agglutination avec des érythrocytes de mouton sensibilisées à l'H.C.G., une sensibilité de 100 UI/l de liquide analysé. 50 μl de la solution d'anticorps, contenant $10^{-3}$ mole diisopropyl fluorophosphate comme inhibiteur de protéases sont introduits dans les tubes de sérologie et lyophilisés. Pour une détermination d'H.C.G., les tubes ont reçu chacun une dose déterminée d'H.C.G. dans 0,15 ml d'urine. Certains tubes ont été laissés à température ambiante pendant quelques heures, et, à des intervalles déterminés, des globules rouges sensibilisés à l'H.C.G. ont été introduits dans les tubes. Les globules rouges étaient suspendus dans 0,25 ml de réactif.

Le tableau ci-dessous illustre le niveau de sensibilité atteint au cours du temps.

3

Tableau I

| H.C.G. dans urine UI/l | Moment de l'addition de cellules indicatrices, en heures après le début de la réaction primaire | | | | | | | |
|---|---|---|---|---|---|---|---|---|
| | 0 | 0,5 | 1 | 2 | 3 | 5 | 7 | 16 |
| 200 | 1 | — | — | — | — | — | — | — |
| 100 | 3 | 3 | 2 | 1 | 1 | 1 | 1 | — |
| 50 | 4 | 3 | 3 | 2 | 2 | 2 | 2 | — |
| 25 | 4 | 4 | 3 | 3 | 3 | 3 | 3 | — |
| 20 | — | — | — | — | — | — | — | 2 |
| 12,5 | 4 | 4 | 4 | 4 | 3 | 3 | 3 | — |
| 10 | — | — | — | — | — | — | — | 3 |
| 5 | — | — | — | — | — | — | — | 3 |
| 2,5 | — | — | — | — | — | — | — | 4 |
| 1,25 | — | — | — | — | — | — | — | 4 |
| 0 | 4 | 4 | 4 | 4 | 4 | 4 | 4 | 4 |

Dans ce tableau, une réaction d'inhibition de l'agglutination, c'est-à-dire la détection de la présence d'H.C.G. dans le liquide analysé, se traduit par des chiffres allant de 0 à 3, zéro signifiant une inhibition intense, et 3 une inhibition faible. Une réaction négative, c'est-à-dire l'absence d'H.C.G. notée par 4, qui signifie donc agglutination complète.

Il est observé que le test IHA, utilisé sous sa forme usuelle c'est-à-dire avec les globules rouges indicateurs ajoutés au moment de la mise en route de la réaction immunologique primaire, au temps O, est sensible à 100 UI H.C.G. par litre d'urine. Cette sensibilité est presque la meilleure qui se puisse obtenir pour un tel test. La sensibilité du test augmente de façon significative lorsque l'addition du système indicateur est retardée.

Une sensibilité 20 fois supérieure est atteinte lorsque la réaction antigène-anticorps primaire en phase liquide est continuée durant toute la nuit. Le niveau de sensibilité alors atteint est celui atteint par les radio-immuno-essais.

Le coût de la détermination de β-H.C.G. par ce nouveau test IHA, le temps nécessaire pour le pratiquer, le niveau de compétence des utilisateurs, sont tous des facteurs qui se comparent de façon très favorable aux tests radio-immunologiques et enzymo-immunologiques.

Exemple 2

Des anticorps furent obtenus contre la sous-unité de l'H.C.G., qui est identique à la sous-unité $\alpha$ de la L.H. 50 $\mu$l d'une dilution adéquate des anticorps contenant $10^{-3}$ mole diisopropyl fluorophosphate (DFP) et 0,4 % EDTA sont lyophilisés dans des tubes de sérologie.

Après lyophilisation, les tubes ont reçu 0,15 ml d'urine mâle contenant des quantités variables de L.H. ou d'H.C.G. L'incubation a eu lieu durant 18 h à température ambiante, après quoi 0,25 ml d'une suspension de globules rouges sensibilisés ont été ajoutés à tous les tubes.

Une sédimentation de cellules a eu lieu durant 2 h et les résultats relevés. Ceux-ci sont condensés dans le tableau II, où 4 signifie complète agglutination, c'est-à-dire absence d'antigène dans le liquide analysé, et de 3 à 0 signifie concentration croissante d'antigène dans le liquide analysé.

Tableau II

| Concentration d'H.C.G. en UI/L | Sensibilité | Concentration de L.H. en UI/L | Sensibilité |
|---|---|---|---|
| 50 | 2 | 100 | 2 |
| 25 | 2 | 50 | 3 |
| 12,5 | 3 | 25 | 4 |
| 6,25 | 4 | 12,5 | 4 |

Le test est plus sensible à l'H.C.G. qu'au L.H. Une concentration en L.H. dans le liquide analysé est détectée sans ambiguïté jusqu'au niveau de 50 UI/l, et le test est sensible à 25 UI par litre. Cette extrême sensibilité rend le test applicable à la détection de l'ovulation.

**0 074 890**

Exemple 3

Des bactéries E. Coli furent sensibilisées par l'H.C.G. Des anticorps anti-H.C.G. furent obtenus chez les lapins par inoculation d'H.C.G. et saignage 14 jours après une inoculation de rappel.

Une dilution d'anticorps anti-H.C.G. fut préparée qui, contenue dans 50 μl de diluant complété par 10⁻³ mole DFP et 0,4 % EDTA, était suffisante pour obtenir une inhibition de l'agrégation des bactéries sensibilisées à l'H.C.G. suspendues dans 50 μl de diluant (0,1 M glycine, pH 8,0 contenant 1 % d'albumine bovine et 0,1 % d'azide de sodium) lorsque cette suspension était mélangée à 50 μl d'urine contenant 500 UI/HCG par litre. A une concentration plus faible de H.C.G. dans l'urine, les bactéries étaient agrégées par les anticorps. 50 μl de la solution d'anticorps furent distribués dans des tubes de sérologie et gardés à 4 °C jusqu'au moment de l'emploi.

Pour le test, les solutions d'anticorps furent ramenés à température ambiante ; 50 μl d'urine contenant différentes concentrations d'H.C.G. variant de 500 à 62,5 UI par litre furent ajoutés à la solution contenue dans les tubes de sérologie et laissés à température ambiante. A des intervalles déterminés, 50 μl de la suspension bactérienne sensibilisée à l'H.C.G. furent ajoutés aux tubes contenant le mélange antigène-anticorps et la totalité du mélange (0,15 ml) fut alors immédiatement transférée sur un porte-objet en verre où le liquide fut maintenu en agitation durant 3 min. par lente rotation de la plaquette.

Après ce temps, une évaluation de l'agrégation ou de l'inhibition de l'agrégation fut effectuée. Un test positif, c'est-à-dire la présence d'H.C.G. soluble, fut noté par une inhibition de l'agrégation et un test négatif, c'est-à-dire absence d'H.C.G. soluble, fut noté par une agrégation de la suspension bactérienne. Le tableau III résume les résultats de cette expérience.

Tableau III

| H.C.G. en UI/l | 0 min. | 15 min. | 30 min. | 45 min. | 60 min. |
|---|---|---|---|---|---|
| 500 | pos. | pos. | pos. | pos. | pos. |
| 250 | neg. | pos. | pos. | pos. | pos. |
| 125 | neg. | neg. | neg. | doute | pos. |
| 62,5 | neg. | neg. | neg. | neg. | neg. |
| 0 | neg. | neg. | neg. | neg. | neg. |

Ce tableau montre que le délai dans l'addition de la phase solide indicatrice à un mélange antigène-anticorps favorise une plus grande sensibilité de ce test. Un délai de 15 min. double la sensibilité et un délai de 60 min. augmente la sensibilité de quatre fois.

Pour les applications pratiques, plusieurs types de conditionnement sont prévus.

Les anticorps spécifiques et les cellules indicatrices pourront être fournies sous forme liquide et en suspension dans deux flacons distincts contenant des inhibiteurs de protéases et des agents bactéricides et bactériostatiques. Les dilutions et la répartition des liquides nécessaires pour obtenir une sensibilité optimale sont alors laissés au soin de l'utilisateur et ce type de conditionnement restreint donc l'emploi des tests aux laboratoires d'analyses médicales.

Des anticorps prédilués au niveau adéquat peuvent être distribués dans des tubes de sérologie. Ils peuvent de surcroît être lyophilisés pour prolonger leur stabilité. La répartition des anticorps dans des proportions définies est préférée puisque la concentration en anticorps doit être correctement ajustée pour l'obtention de bons résultats. Avec un pareil conditionnement individuel, il suffit alors d'ajouter au tube une quantité déterminée de liquide à analyser, attendre que la réaction immunologique primaire ait eu lieu, puis ajouter le système indicateur.

Si le test est destiné au grand public, un conditionnement préférentiel est la fourniture de tubes de sérologie contenant des anticorps prédilués sous forme lyophilisée. Si le système indicateur est composé de globules rouges sensibilisées, les tubes sont insérés au-dessus d'un miroir et les globules rouges sont contenus dans une pochette plastique. Le nombre de pochettes contenant le système indicateur est égal à celui des tubes fournis.

L'utilisateur doit alors :

1. Ajouter 2 à 3 gouttes d'urine à un tube.
2. Attendre 5 à 7 heures dépendant du niveau de sensibilité désiré.
3. Ajouter le contenu d'une pochette au même tube.
4. Attendre 2 heures et lire dans le miroir le type de sédimentation obtenu.

**Revendications**

1. Un procédé pour la détection d'antigènes et d'anticorps par un test d'inhibition d'agglutination,

5

selon un mécanisme immunologique du type réaction antigène-anticorps, comprenant la réaction des anticorps avec les antigènes en phase liquide dans des conditions de réaction entre lesdits anticorps et lesdits antigènes pour réaliser une réaction immunologique primaire, et l'ajout d'un système indicateur approprié, caractérisé en ce que ledit système indicateur est un système indicateur sensibilisé que l'on ajoute avec un retard pouvant aller de 15 minutes, de préférence 1 heure, à 18 heures après que la réaction antigènes-anticorps ait eu lieu.

2. Procédé selon la revendication 1, caractérisé par le fait que le système indicateur sensibilisé est une phase solide, granulée, sensibilisée.

3. Procédé selon la revendication 1 ou 2, caractérisé par le fait que le système indicateur sensibilisé est une phase solide granulée sensibilisée à l'antigène.

4. Procédé selon les revendications 1, 2 et 3 où la phase solide granulée est composée d'érythrocytes sensibilisés.

5. Procédé selon les revendications 1, 2 et 3 où la phase solide granulée consiste en bactéries sensibilisées.

6. Procédé selon la revendication 2, caractérisé par le fait que les anticorps spécifiques sont lyophilisés en quantité déterminée dans des tubes individuels.

7. Procédé selon la revendication 3, caractérisé par le fait que l'antigène est lyophilisé en quantité déterminée dans des tubes individuels.

8. Procédé selon la revendication 1, caractérisé par le fait que le système indicateur solide est conditionné sous forme de suspension en volumes unitaires.

9. Procédé selon la revendication 8, caractérisé par le fait que le système indicateur solide est conditionné sous la forme d'une suspension dans des pochettes plastiques unitaires.

10. Procédé selon les revendications 1, 5 et 6 où la solution antigènes et anticorps contient des agents bactéricides, bactériostatiques et des inhibiteurs de protéases.

## Claims

1. A process of detecting antigens and antibodies by an agglutination inhibiting test, according to an immunological mechanism of the antigen-antibody reaction type, including a reaction of the antibodies with the antigens in a liquid phase under conditions of reaction between the said antibodies and the said antigens in order to perform a primary immunological reaction, and the addition of an appropriate indicating system, characterized in that the said indicating system is a sensitized indicating system which is added with a delay which may range from 15 minutes, preferably one hour, to 18 hours after the antigen-antibody reaction has taken place.

2. A process according to claim 1, characterized by the fact that the sensitized indicating system is a sensitized solid, granulated phase.

3. A process according to claim 1 or 2, characterized by the fact that the sensitized indicating system is a solid, granulated phase made sensitive to the antigen.

4. A process according to claims 1, 2 and 3 wherein the solid, granulated phase is constituted by sensitized erythrocytes.

5. A process according to claims 1, 2 and 3, wherein the solid, granulated phase consists of sensitized bacteria.

6. A process according to claim 2, characterized by the fact that the specific antibodies are lyophilized in a given quantity in individual tubes.

7. A process according to claim 3, characterized by the fact that the antigen is lyophilized in a given quantity in individual tubes.

8. A process according to claim 1, characterized by the fact that the solid indicating system is packaged in the form of a suspension in unit volumes.

9. A process according to claim 8, characterized by the fact that the solid indicating system is packaged in the form of a suspension in unit packets of plastics material.

10. A process according to claims 1, 5 and 6, wherein the antigen and antibody solution contains bactericidal, bacteriostatic agents and protease inhibitors.

## Patentansprüche

1. Ein Verfahren zum Nachweisen von Antigenen und Antikörpern durch einen Agglutinationshemmungstest gemäss einem Immunitätsmechanismus der Antigen-Antikörperreaktionsart, umfassend die Reaktion der Antikörper mit den Antigenen in flüssiger Phase unter Reaktionsverhältnissen zwischen den besagten Antikörpern und den besagten Antigenen, um eine Primärimmunitätsreaktion durchzuführen, und die Zufügung eines geeigneten Anzeigesystems, dadurch gekennzeichnet, dass das besagte Anzeigesystem ein sensibilisiertes Anzeigesystem ist, welches man mit einer Verspätung von etwa 15 Minuten, vorzugsweise einer Stunde, bis 18 Stunden nachdem die Antigenen-Antikörperreaktion stattgefunden hat, zutut.

2. Verfahren nach Anspruch 1, gekennzeichnet durch die Tatsache, dass das sensibilisierte Anzeigesystem eine sensibilisierte, gekörnte, Feststoff-Phase ist.

3. Verfahren nach Anspruch 1 oder 2, gekennzeichnet durch die Tatsache, dass das sensibilisierte Anzeigesystem eine mit dem Antigensensibilisierte, gekörnte, Feststoff-Phase ist.

4. Verfahren nach den Ansprüchen 1, 2 und 3, wobei die gekörnte Feststoff-Phase aus sensibilisierten Erythrocyten besteht.

5. Verfahren nach den Ansprüchen 1, 2 und 3, wobei die gekörnte Feststoff-Phase aus sensibilisierten Bakterien besteht.

6. Verfahren nach Anspruch 2, gekennzeichnet durch die Tatsache, dass die spezifischen Antikörper in bestimmter Menge in Einzelrohren lyophilisiert sind.

7. Verfahren nach Anspruch 3, gekennzeichnet durch die Tatsache, dass das Antigen in bestimmter Menge in Einzelrohren lyophilisiert ist.

8. Verfahren nach Anspruch 1, gekennzeichnet durch die Tatsache, dass das Feststoff-Anzeigesystem als Aufschwemmung in Einheitsvolumen aufbereitet ist.

9. Verfahren nach Anspruch 8, gekennzeichnet durch die Tatsache, dass das Feststoff-Anzeigesystem als Aufschwemmung in Einheitskunststofftäschen aufbereitet ist.

10. Verfahren nach Ansprüchen 1, 5 und 6, wobei die Lösung aus Antigenen und Antikörpern Bakterizid-, bakteriostatische und Proteasehemmende Mittel enthält.